(19) Europäisches Patentamt
European Patent Office
Office européen des brevets

(11) Publication number: **0 023 093**
**B1**

(12) **EUROPEAN PATENT SPECIFICATION**

(45) Date of publication of patent specification: 28.09.83

(21) Application number: 80302152.6

(22) Date of filing: 26.06.80

(51) Int. Cl.[3]: A 61 K 31/43,
A 61 K 31/545,
C 07 D 499/00 //(A61K31/43, 31/545)

(54) **Penicillanic acid derivatives, their preparation and their pharmaceutical compositions with a penicillin or cephalosporin.**

(30) Priority: 02.07.79 GB 7922885

(43) Date of publication of application:
28.01.81 Bulletin 81/4

(45) Publication of the grant of the patent:
28.09.83 Bulletin 83/39

(84) Designated Contracting States:
CH DE FR GB IT LI NL

(56) References cited:
FR - A - 2 457 213
NL - A - 8 002 843
US - A - 4 143 046

(73) Proprietor: BEECHAM GROUP PLC
Beecham House Great West Road
Brentford Middlesex (GB)

(72) Inventor: Harbridge, John Barry
109 St Andrews Road
Coulsdon Surrey (GB)
Inventor: Denerley, Paul Millington
45 Avondale Close
Horley Surrey (GB)

(74) Representative: Hesketh, Alan, Dr. et al,
European Patent Attorney Beecham
Pharmaceuticals Great Burgh Yew Tree Bottom
Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 023 093 B1

# Penicillanic acid derivatives, their preparation and their pharmaceutical compositions with a penicillin or cephalosporin

This invention relates to a class of penicillanic acid derivatives which are useful as β-lactamase inhibitors.

Certain 6β-sulphonyloxypenicillanic acids are known [Sheehan *et al.* Cambridge symposium on β-lactam antibiotics June 1976; and US—A—4 143 046]. However in the past these compounds have only been disclosed as having antibacterial activity, which has proved disappointing. No mention has been made that any of these compounds might possess β-lactamase inhibitory properties. Surprisingly, it has been found that these and related compounds possess the ability to enhance the effectiveness of penicillins and cephalosporins.

The present invention provides a pharmaceutical composition comprising:

(a) a pharmaceutically acceptable carrier;

(b) a compound of the formula (I):

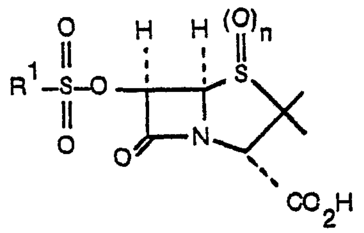

(I)

and pharmaceutically acceptable salts and in vivo hydrolysable esters thereof, wherein n is 0 or 2, $R^1$ is a $C_{1-6}$ alkyl group optionally substituted with one, two or three fluorine, chlorine or bromine atoms, or is a di-$C_{1-6}$ alkylamino group or is a group of the formula (i):

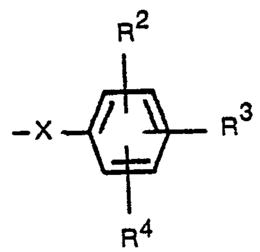

(i)

wherein

X is a bond, or a —CH=CH group, or a methylene or ethylene group; $R^2$ is a hydrogen, fluorine, bromine or chlorine atom, or is an amino, protected amino, hydroxy, protected hydroxy, $C_{1-6}$ alkyl, nitro, di-$C_{1-6}$ alkylamino, acetamido, $C_{1-6}$ alkoxy or trifluoromethyl group; $R^3$ is a hydrogen or chlorine atom or a $C_{1-6}$ alkyl or trifluoromethyl group; and $R^4$ is a hydrogen or chlorine atom, or a $C_{1-6}$ alkyl or $C_{1-6}$ alkoxy group; or $R^2$ and $R^3$ on any two adjacent carbon atoms may together represent a buta-1,3-dienylene moiety which is optionally substituted by a $C_{1-3}$ alkyl or $C_{1-3}$ alkoxy group; and

(c) a penicillin or cephalosporin or a pharmaceutically acceptable salt or in vivo hydrolysable ester thereof.

When the terms "protected amino" and "protected hydroxy" are used in this specification, the groups used herein for protecting these functions are those that may be removed when desired; for example any of such group known in the art, such as a benzyloxycarbonyl, p-nitrobenzyloxycarbonyl or benzyl group.

Preferably n is zero.

Suitably $R^1$ is a $C_{1-6}$ alkyl group optionally substituted with one, two or three fluorine, chlorine or bromine atoms.

More suitably $R^1$ is a methyl, ethyl, propyl, chloropropyl or isopropyl group.

In compounds of the formula (i) suitably X is a bond or a methylene group.

Apt values for $R^2$ include the hydrogen, fluorine, chlorine and bromine atoms, and amino, protected amino, hydroxy, protected hydroxy, $C_{1-6}$ alkyl, nitro, $C_{1-6}$ alkylamino, acetamido, $C_{1-6}$ alkoxy and trifluoromethyl groups.

Suitably $R^2$ is a hydrogen, fluorine, bromine or chlorine atom, or a methyl or methoxy group.

A preferred value of $R^2$ is methyl.

A further preferred value of $R^2$ is bromine.

Apt values for $R^3$ are a hydrogen or chlorine atom, or a $C_{1-6}$ alkyl or trifluoromethyl group.

Suitably $R^3$ is a hydrogen or chlorine atom.

A preferred value for $R^3$ is hydrogen.

Apt values for $R^4$ are a hydrogen or chlorine atom, or a $C_{1-6}$ alkyl group.

Suitably $R^4$ is a hydrogen or chlorine atom.

A preferred value for $R^4$ is hydrogen.

Thus it is to be realised that a particularly suitable sub-group of the compounds for use in the compositions of this invention is that of the formula (II):

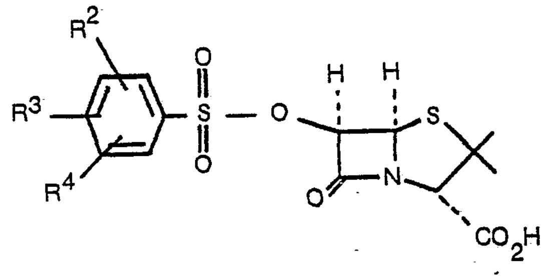

(II)

and pharmaceutically acceptable salts and in-vivo hydrolysable esters thereof, wherein $R^2$, $R^3$ and $R^4$ are as hereinbefore defined.

Preferably in the compounds of the formula (II) $R^3$ and $R^4$ are both hydrogen atoms and $R^2$ is selected from hydrogen, bromine, fluorine, chlorine, $C_{1-6}$ alkyl nitro and amino. Most favourably $R^3$ and $R^4$ are both hydrogen and $R^2$ is hydrogen, methyl, bromine or amino.

Preferably also in the compounds of the formula (II) $R^2$ and $R^3$ are on any two adjacent carbon atoms and represent a buta-1,3-dienylene moiety, thus forming a naphthyl group.

Suitably also in the compounds of the formula (II) $R^2$, $R^3$ and $R^4$ are all methyl groups.

The compounds of the formulae (I)—(II) are suitably provided in the form of the free acid. Alternatively the compounds of the formulae (I)—(II) are provided in the form of a pharmaceutically acceptable salt.

Examples of pharmaceutically acceptable salts of the compounds of the formulae (I)—(II) include the alkali metal salts such as sodium or potassium, alkaline earth metal salts such as calcium or magnesium, and ammonium or substituted ammonium salts for example those with lower alkylamines such as triethylamine, hydroxy-lower alkylamines such as 2-hydroxyethylamine, bis-(2-hydroxyethyl)-amine, tris-(hydroxymethyl)amine or tris-(2-hydroxyethyl)-amine, cycloalkylamines such as bicyclohexylamine, or with procaine, dibenzylamine, N,N-dibenzylethylenediamine, 1-ephenamine, N-ethylpiperidine, N-benzyl-$\beta$-phenethylamine, dehydroabietylamine or N,N'-bis-dehydroabietylethylenediamine.

Thus suitable salts of this invention include the sodium, potassium, calcium, magnesium and ammonium salts, of these the sodium, potassium and calcium salts are favoured.

The compounds of the formulae (I)—(II) may be provided as in-vivo hydrolysable esters. Such esters are those which hydrolyse in the human body to produce the parent acid. Suitable in-vivo hydrolysable esters include those esters known to give in-vivo hydrolysis in penicillins. Thus suitable esters include those of the formula (ii):

$$—CO—O—CHR_1—O—CO—R_2 \qquad \text{(ii)}$$

wherein

$R_1$ is a hydrogen atom, or a methyl or phenyl group; $R_2$ is an alkyl group of 1 to 6 carbon atoms, a phenyl group, an alkyl group of 1 to 3 carbon atoms substituted by a phenyl group, an alkoxy group of 1 to 6 carbon atoms, a phenoxy group, or an alkoxy group of 1 to 3 carbon atoms substituted by a phenyl group; or $R_1$ is attached to $R_2$ to form a 1,2-diphenylene or 4,5-dimethoxy-1,2-diphenylene group.

Favourably $R_1$ is hydrogen.

When $R_1$ is hydrogen suitably $R_2$ is selected from methyl, ethyl, n-propyl, iso-propyl, n-butyl, tert-butyl, phenyl, benzyl, methoxy, ethoxy, n-propyloxy and isopropyloxy. Preferably $R_2$ is tert-butyl.

Favourably $R_1$ and $R_2$ are joined so that the ester is a phthalidyl or 3,4-dimethoxyphthalidyl ester.

Of these esters those favoured as in-vivo hydrolysable esters are the acetoxymethyl, acetoxyethyl, phthalidyl, ethoxycarbonyloxymethyl, $\alpha$-ethoxycarbonyloxyethyl and pivaloyloxymethyl esters.

The in-vivo hydrolysable nature of the ester may be confirmed by administration to an animal such as a mouse or rat and determination of the presence of a compound of the formula (I) or a salt thereof in the blood or urine of the animal. Alternatively hydrolysis in human blood or serum may be determined.

It must be realised that salts of the compounds of the formulae (I)—(II) formed with pharmaceutically unacceptable ions are useful as they may serve as intermediates in the preparation of pharmaceutically acceptable salts by ion-exchange, or they may be useful as intermediates in the

preparation of in-vivo hydrolysable esters. An example of such a salt is the lithium salt.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

This invention also provides the use of a penicillin or cephalosporin or pharmaceutically acceptable salt or in-vivo hydrolysable ester thereof in composition with a compound of the formula (I) or pharmaceutically acceptable salt or in-vivo hydrolysable ester thereof, for preparing injectable aqueous solutions.

Such solutions may be prepared by dissolving the sterile compound of this invention and the penicillin or cephalosporin in sterile water. Suitably this water is "Water for Injection BP" or the equivalent and may contain electrolytes to render it isotonic.

A particularly suitable injectable aqueous solution of this invention is one which contains not less than 15% w/w of the salt of the compound of the formula (I).

Unit dose compositions comprising a compound of this invention adapted for oral administration form a further suitable composition aspect of this invention.

Orally administrable compositions are of use as a synergistically effective blood level can be expected at high dose and at lower doses such compositions may be used to treat infections localised in the gastro-intestinal tract.

Unit dose compositions comprising a compound of this invention adapted for topical administration are also presented by this invention. In this instance 'topical administration' also includes local administration to internal surfaces of mammary glands of cattle, for example during the treatment of mastitis by intramammary administration. Considerable advantages accrue from the inclusion of a penicillin or cephalosporin since the resulting composition shows enhanced effectiveness (synergy).

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin and other known penicillins including pro-drugs therefore such as their in-vivo hydrolysable esters such as the acetoxymethyl, pivaloyloxymethyl, $\alpha$-ethoxycarbonyloxyethyl or phthalidyl esters of ampicillin, benzylpenicillin or amoxycillin, and aldehyde or ketone adducts of penicillins containing a 6-$\alpha$-aminoacetamide side chain (such as hetacillin, metampicillin and analogous derivatives of amoxycillin) or $\alpha$-esters of carbenicillin or ticarcillin such as their phenyl or indanyl $\alpha$-esters.

Suitable cephalosporins for inclusion in the compositions of this invention include cefatrizine, cephaloridine, cephalothin, cefazolin, cephalexin, cephacetrile, cephamandole nafate, cephapirin, cepradine, 4-hydroxycephalexin, cefaprole, cephaloglycin, and other known cephalosporins or pro-drugs thereof.

Such compounds are frequently used in the form of a salt for example the sodium, potassium or calcium salt or a hydrate thereof.

Naturally if the penicillin or cephalosporin present in the composition is not suitable for oral administration then the composition will be adapted for parenteral administration. As previously indicated such injectable or infusable compositions can be particularly apt.

Highly favoured penicillins for use in the compositions of this invention include ampicillin, amoxycillin, carbenicillin and ticarcillin. Such penicillins may be used as a pharmaceutically acceptable salt such as the sodium salt.

Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a composition of this invention.

The preferred penicillin for use in the synergistic composition is amoxycillin, for example as its sodium salt or trihydrate.

Particularly suitable cephalosporins for use in the compositions of this invention include cephaloridine and cefazolin.

When present together with a cephalosporin or penicillin, the ratio of a compound of the invention to the penicillin or cephalosporin agent may vary over a wide range of ratios, such as from 10:1 to 1:10

for example about 3:1, 2:1, 1:1, 1:2, 1:3, 1:4, 1:5 or 1:6, (wt/wt, based on pure free antibiotic equivalent).

The total quantity of a compound of the formula (I) or its pharmaceutically acceptable salt or in vivo hydrolysable ester in any unit dosage will normally be between 25 and 1000 mg and will usually be between 50 and 500 mg, for example about 62.5, 100, 125, 150, 200 or 250 mg.

Compositions of this invention may be used for the treatment of infections inter alia, the respiratory tract, the urinary tract and soft tissues in humans and mastitis in cattle.

Normally between 50 and 1000 mg of a synergist will be administered each day of treatment but more usually as 2, 3 or 4 doses.

The penicillin or cephalosporin in the synergistic composition of this invention will normally be present at approximately the amount at which it is conveniently used which will usually be expected to be from about 62.5 to 1000 mg per dose, more usually about 125, 250 or 500 mg per dose.

One particularly favoured composition of this invention will contain from 150 to 1000 mg of amoxycillin as the trihydrate or sodium salt and from 25 to 500 mg of a synergist.

Most suitably this form of composition will contain a pharmaceutically acceptable salt of the compound of the formula (I).

A further particularly favoured composition of this invention will contain from 150 to 1000 mg of ampicillin or a pro-drug therefor and from 25 to 500 mg of a synergist.

Most suitably this form of composition will contain ampicillin trihydrate, ampicillin anhydrate, sodium ampicillin, hetacillin, pivampicillin hydrochloride bacampicillin hydrochloride or talampicillin hydrochloride. Most suitably this form of the composition will contain a compound of the formula (I) or its pharmaceutically acceptable salt.

Most suitably the preceding compositions will contain from 200 to 700 mg of the penicillin component. Most suitably the preceding composition will comprise from 50 to 250 mg of a compound of the formula (I).

Such compositions may be adapted for oral or parenteral use except when containing an in vivó hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a synergist.

Suitably this form of composition will contain di-sodium carbenicillin. Suitably this form of the composition will contain di-sodium ticarcillin.

More suitably this form of the composition will contain from 75 to 250 mg of a compound of the formula (I) or pharmaceutically acceptable salt or in vivo hydrolysable ester thereof preferably in crystalline form. Such compositions containing di-salts of carbenicillin and ticarcillin will be adapted for parenteral administration.

Commonly the infection treated will be due to a strain of *Staphylococcus aureus, Escherichia coli* or *Proteus sp.* The organisms named are readily treated by using a synergistically effective amount of the synergist and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides the compounds of the formula (I) or pharmaceutically acceptable salts or in vivo hydrolysable esters thereof with the proviso that when n is 0, then $R^1$ is not methyl, benzyl, p-chlorobenzyl, phenyl or p-aminophenyl when the compounds of this invention are in the form of a free acid or a pharmaceutically acceptable salt.

Suitable classes of groups and novel compounds of this invention are those described above in relation to the compounds of the formula (I) and (II) for use in the compositions of this invention.

The compounds of the formula (I) or salts or esters thereof may be prepared by the reaction of a compound of the formula (III), or a derivative thereof which allows acylation to take place:

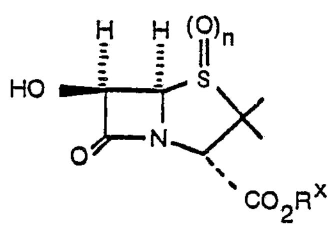

(III)

wherein

n is 0 or 2 and $R^x$ is a hydrogen atom or a carboxy protecting group, with an O-sulphonating derivative of a compound of the formula (IV):

$R^1SO_3H$ (IV)

wherein

$R^1$ is as hereinbefore defined in relation to formula (I); and thereafter if desired,

a) cleaving the carboxy protecting group to form the free acid or salt thereof.

b) converting the free acid or salt into a pharmaceutically acceptable salt or in-vivo hydrolysable ester,

c) converting the compound wherein n is 0 to the compound wherein n is 2.

Suitable O-sulphonating derivatives include $R^1SO_2Cl$, $R^1SO_2Br$ and $(R^1SO_2)_2O$; of these the sulphonyl chloride is preferred.

Suitable groups $R^x$ include the hydrogen atom, in-vivo hydrolysable esters as hereinbefore described, and conventional protecting groups well-known to those skilled in the art of penicillin chemistry. Such conventional protecting groups include salts and ester derivatives of the carboxylic acid.

The derivative is preferably one which may readily be cleaved at a later stage of the reaction. Suitable salts include sodium, potassium, dicyclohexylamine and tertiary amine salts, such as those with tri-lower-alkylamines, N-ethylpiperidine, 2,6-lutidine, pyridine, N-methylpyrrolidine, dimethylpiperazine. A preferred salt is with triethylamine.

Suitable ester-forming carboxyl-protecting groups are those which may be removed under conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetrahydrofuran-2-yl, tetrahydropyran-2-yl, pentachlorophenyl, p-toluenesulphonylethyl, methoxymethyl, a silyl group such as trimethylsilyl, a stannyl or phosphorus-containing group, or an oxime radical of formula —N=CHR° where R° is aryl or heterocyclic.

The carboxyl group may be regenerated from any of the above esters by usual methods appropriate to the particular $R^x$ group, or by enzymatically — catalysed hydrolysis, or by hydrogenation.

Suitably the reaction of a compound of the formula (III) with a compound of the formula (IV) is performed in the presence of an organic or inorganic base. More suitably the base is an organic amine such as a tri-$C_{1-6}$ alkylamine or pyridine. Preferably the base is triethylamine. Inorganic bases may be used for example sodium hydroxide, potassium hydroxide or sodium hydride.

Suitably the reaction of an ester of a compound of the formula (III) and a compound of the formula (IV) as hereinbefore described is performed in an inert organic solvent, for example diethylether, tetrahydrofuran, dioxan, 1,2-dimethoxyethane, chloroform, dichloromethane, ethyl acetate, acetone and acetonitrile.

A particularly suitable solvent is dichloromethane.

The reaction of an inorganic salt of a compound of the formula (III) such as the sodium or potassium salt and a compound of the formula (IV) is suitably performed in an aqueous solvent system. The reaction of an organic amine salt of a compound of the formula (III) and a compound of the formula (IV) is suitably performed in a chlorinated solvent, for example dichloromethane.

Suitably the reaction is performed at a temperature between −30° and 30°. A particularly preferred procedure is to commence the reaction at 0°C and permit the reaction temperature to rise slowly to ambient.

Since it is frequently desirable to form a salt of the compound of the formula (I), it is a preferred embodiment to use as the carboxyl protecting group in the compounds of the formula (III) an ester that is readily converted to the parent acid or its salt by mild methods of hydrogenolysis. Particularly suitable esters for use in this process include benzyl esters optionally substituted by $C_{1-6}$ alkyl, $C_{1-6}$ alkoxy, bromine, chlorine or nitro. A preferred ester is the benzyl ester.

Suitable methods of hydrogenation include hydrogenation in the presence of a transition metal catalyst. The pressure of hydrogen used in the reaction may be low, medium or high but in general an approximately atmospheric or slightly super-atmospheric pressure of hydrogen is preferred. The transition metal catalyst employed is preferably palladium on charcoal or on calcium carbonate. The hydrogenation may be effected in any inert solvent in which the ester is soluble for example tetrahydrofuran, ethyl acetate, methanol, ethanol, and mixtures thereof together with water. If this hydrogenation is carried out in the presence of a base then a salt of the compound of the formula (I) is produced. Suitable bases for inclusion include $NaHCO_3$, $KHCO_3$, $Na_2CO_3$, $CaCO_3$, $MgCO_3$, $LiHCO_3$, $NH_4OCOCH_3$ and the like. If no base is present then hydrogenation leads to the preparation of an acid within formula (I) which may then be neutralised if desired to yield a salt. Suitable bases which may be used to neutralise acids within formula (I) include LiOH, NaOH, $NaHCO_3$, KOH, $Ca(OH)_2$ and $Ba(OH)_2$.

The salts of acids (I) may be converted to esters in conventional manner, for example by reaction with a reactive halide such as bromomethyloxy pivalate in solution in dimethylformamide.

The substituent group or groups within the groups $R_2$ and $R_3$ in the compounds of formula (I) may be varied by conventional reactions. Thus for example when a substituent is a nitro group it may be reduced in a conventional manner to an amino group, for example by catalysed hydrogenation. Similarly an amino group may be acylated to give a substituted amino group, for example by treatment

with an acyl halide in the presence of an organic base. Substituents such as $NHCO_2$p-nitrobenzyl of $OCO_2$p-nitrobenzyl may be converted to an amino or hydroxyl group respectively for example by hydrogenolysis.

The compounds of the formula (III) and derivatives thereof wherein n is 0 are described in United States Patent No. 4,143,046 which is herein incorporated by reference.

The compounds of formula (I) wherein n is 2, and pharmaceutically acceptable salts and in vivo hydrolysable esters thereof may be prepared by treatment of the corresponding compound of the formula (I) wherein n is 0 with an oxidising agent in an inert solvent at a non-extreme temperature, for example 0°—30°C.

A suitable oxidising agent is m-chloroperbenzoic acid.

When the oxidising agent used is m-chloroperbenzoic acid suitable solvents include water, diethyl ether, tetrahydrofuran, dioxan, ethyl acetate, chloroform and dichloromethane. Preferred solvents are dichloromethane, diethyl ether and chloroform.

In analogous manner the compounds of the formula (III) wherein n is 2 may be prepared from compounds of the formula (III) wherein n is 0.

The following Examples illustrate the invention.

Example 1

Calcium 6β-p-bromobenzenesulphonyloxypenicillanate

Benzyl 6β-hydroxypenicillanate (0.202 g) was dissolved in chloroform (3 ml) containing dimethylformamide (0.15 ml), and to this stirred solution was added p-bromobenzenesulphonyl chloride (0.255 g) and triethylamine (0.092 ml). After 24 hours the reaction mixture was diluted with chloroform (20 ml), washed with dilute sodium bicarbonate (15 ml), washed with water (15 ml), dried ($MgSO_4$), and evaporated in vacuo to afford a gum. This gum was subjected to rapid column chromatography on silica gel (20 g) using ethyl acetate:cyclohexane (1:3). The appropriate fractions were combined and evaporated in vacuo to afford an oil, which was crystallised from ethyl acetate-cyclohexane to yield benzyl 6β-p-bromobenzenesulphonyloxypenicillanate as white crystals, m.p. 117—118°C; i.r. (Nujol) 1795, 1755, 1745 $cm^{-1}$; n.m.r. ($CDCl_3$) 1.36 (3H, s), 1.56 (3H, s), 4.45 (1H, s), 5.15 (2H, s), 5.47 (1H, d $J$ = 4Hz), 5.65 (1H, d $J$ = 4Hz), 7.35 (5H, s), 7.77 (4H, ABq $J$ = 9Hz).

Benzyl 6β-p-bromobenzenesulphonyloxypenicillanate (0.097 g) was dissolved in a solvent mixture of tetrahydrofuran (3 ml), methanol (3 ml), and water (3 ml), and was hydrogenated over 5% Palladium on calcium carbonate (0.3 g) for 1 hour. The catalyst was filtered off and washed liberally with water and methanol. The combined filtrates were concentrated in vacuo to afford the title compound as a white solid (0.09 g), i.r. (KBr) 3400, 1778, 1595 $cm^{-1}$; n.m.r. ($D_2O$) 1.38 (3H, s), 1.49 (3H, s), 4.19 (1H, s), 5.37 (1H, d $J$ = 4.5Hz), 5.87 (1H, d $J$ = 4.5Hz), 7.5—8.1 (4H, m).

Example 2

Sodium 6β-ethanesulphonyloxypenicillanate

To a solution of benzyl 6β-hydroxypenicillanate (0.5 g) in dichloromethane (10 ml) containing triethylamine (0.4 ml) at −10°C was added ethanesulphonyl chloride (0.2 ml). The solution was stirred at −10°C for 15 min., then washed with iced water, dilute hydrochloric acid, sodium bicarbonate, brine and finally dried over anhydrous magnesium sulphate. Removal of the solvent in vacuo yielded benzyl 6β-ethanesulphonyloxypenicillanate as a pale yellow oil (0.57 g), $\nu_{max}$ ($CHCl_3$) 1800, 1750, 1500, 1460, 1370, 1300, 1280, 1180, 850 and 840 $cm^{-1}$; $[\alpha]_D^{20}$ + 173.3° (c 1.2% $CHCl_3$), $\delta$ ($CDCl_3$) 7.35 (5H, s, Ar$H$), 5.75 (1H, d, $J$ = 3.7Hz, $H$—$C_6$), 5.52 (1H, d, $J$ = 3.7Hz, $H$—$C_5$), 5.17 (2H, s, $CH_2$—Ph), 4.50 (1H, s, $H$—$C_3$), 3.28 (2H, q, $J$ = 7.4Hz, Me—$CH_2$—S), 1.63 (3H, s, gem $CH_3$), 1.46 (3H, t, $J$—7.4Hz, $CH_3$—$CH_2$—S), 1.43 (3H, s, gem $CH_3$).

Benzyl 6β-ethanesulphonyloxypenicillanate (0.2 g) was dissolved in tetrahydrofuran (10 ml) containing water (3 drops) and 10% palladium-carbon catalyst (0.4 g). The mixture was hydrogenolysed at atmospheric pressure for $\frac{1}{2}$ hr, then filtered (celite) and evaporated in vacuo. The resulting oil was partitioned between ethyl acetate:water and neutralised with sodium hydroxide (0.5 M). The aqueous layer was separated and the water removed in vacuo to yield sodium 6β-ethanesulphonyloxypenicillanate as a pale brown solid (0.108 g), $\nu_{max}$ (KBr) 1750, 1610, 1520, 1430, 1360, 1240, 1170, 890 and 860 $cm^{-1}$; $[\alpha]_D^{20}$ = +219.4° (c 1.2 $H_2O$), $\delta$ ($D_2O$) 5.92 (1H, d, $J$ = 3.7Hz, $H$—$C_6$), 5.61 (1H, d, $J$ = 3.7Hz, $H$—$C_5$), 4.27 (1H, s, $H$—$C_3$), 3.46 (2H, q, Me—$CH_2$—S), 1.56 (3H, s, gem $CH_3$), 1.45 (3H, s, gem $CH_3$), 1.35 (3H, t, $CH_3$—$CH_2$—S).

Example 3

Sodium 6β-(2-naphthalenesulphonyloxy)penicillanate

To a solution of benzyl 6β-hydroxypenicillanate (0.6 g) in pyridine (10 ml) was added 2-naphthalenesulphonyl chloride (0.8 g). The solution was allowed to stand at 4°C for 24 hr., and then poured on to iced water, and extracted with ethyl acetate (2 × 20 ml). The ethyl acetate layer was then washed with dilute sulphuric acid (2 × 40 ml) and dried over anhydrous sodium sulphate — sodium carbonate. Removal of the solvent in vacuo yielded benzyl 6β-(2-naphthalenesulphonyloxy)-penicillanate as a pale yellow oil (0.5 g), $\nu_{max}$ ($CHCl_3$) 1800, 1750, 1460, 1180 and 900 $cm^{-1}$; $\delta$

($CDCl_3$) 7.55 (7H, m, Ar*H*) 7.35 (5H, s, Ar*H* ester), 5.70 (1H, d, *J* = 4Hz, *H*—$C_6$), 5.45 (1H, d, *J* = 4Hz, *H*—$C_5$), 5.15 (2H, s, $CH_2$—Ph), 4.45 (1H, s, *H*—$C_3$), 1.55 (3H, s, gem $CH_3$), 1.35 (3H, s, gem $CH_3$).

Benzyl 6β-(2-naphthalenesulphonyloxy)penicillanate (0.4 g), was dissolved in tetrahydrofuran (15 ml) containing water (3 drops) and 10% palladium — carbon catalyst (0.8 g). The mixture was hydrogenolysed at atmospheric pressure for ½ hr, then filtered (celite) and the solvent evaporated in vacuo. The resulting oil was partitioned between ethyl acetate:water and neutralised with sodium hydroxide (0.5 M). The aqueous layer was separated and freeze dried to yield sodium 6β-(2-naphthalenesulphonyloxy)penicillanate as a white solid, $\omega_{max}$ (KBr), 1785, 1610, 1410, 1390, 1230, 1185 and 900 $cm^{-1}$; δ ($D_2O$) 8.65 (1H, s, Ar*H*), 8.00 (6H, m, $Ar_H$), 6.05 (1H, d, *J* = 3.8Hz, *H*—$C_6$), 5.55 (1H, d, *J* = 3.8Hz, *H*—$C_5$), 4.23 (1H, s, *H*—$C_3$), 1.60 (3H, s, gem $CH_3$), 1.50 (3H, s, gem $CH_3$).

## Example 4

Sodium 6β-mesitylenesulphonyloxypenicillanate

To a solution of benzyl 6β-hydroxypenicillanate (0.64 g) in pyridine (10 ml) was added mesitylenesulphonyl chloride (0.5 g). The solution was allowed to stand at 4°C for 24 hr. then poured on to iced water and extracted with ethyl acetate (2 x 20 ml). The ethyl acetate layer was then washed with dilute sulphuric acid (2 x 20 ml) and dried over anhydrous sodium sulphate — sodium carbonate. Removal of the solvent in vacuo yielded benzyl 6β-mesitylenesulphonyloxypenicillanate (0.8 g), $\nu_{max}$ ($CHCl_3$) 1790, 1740, 1600, 1190 and 880 $cm^{-1}$; δ ($d_6$-DMSO) 7.40 (5H, s, Ar*H*), 7.15 (2H, s, Ar*H*), 5.85 (1H, d, *J* = 3.8Hz, *H*—$C_6$), 5.48 (1H, d, *J* = 3.8Hz, *H*—$C_5$), 5.20 (2H, s, $CH_2$Ph), 4.55 (1H, s, *H*—$C_3$), 2.60 (6H, s, Ar$CH_3$), 2.30 (3H, s, Ar$CH_3$), 1.55 (3H, s, gem $CH_3$), 1.35 (3H, s, gem $CH_3$).

Benzyl 6β-mesitylenesulphonyloxypenicillanate (0.45 g) was dissolved in tetrahydrofuran (15 ml) containing water (3 drops) and 10% palladium-carbon catalyst (0.9 g). The mixture was hydrogenolysed at atmospheric pressure for ½ hr. then filtered (celite) and the solvent evaporated in vacuo. The resulting oil was partitioned between ethyl acetate:water and neutralised with sodium hydroxide (0.5 M). The aqueous layer was separated and freeze dried to yield sodium 6β-mesitylene-sulphonyloxypenicillanate as a white solid (0.39 g), $\nu_{max}$ (KBr), 1780, 1600, 1190 and 850 $cm^{-1}$; δ ($D_2O$) 6.90 (2H, s, Ar*H*), 5.62 (1H, d, *J* = 3.8Hz, *H*—$C_6$), 5.30 (1H, d, *J* = 3.8Hz, *H*—$C_5$), 4.05 (1H, s, *H*—$C_3$), 2.35 (6H, s, Ar$CH_3$), 2.05 (3H, s, Ar$CH_3$), 1.35 (3H, s, gem $CH_3$), 1.25 (3H, s, gem $CH_3$).

## Example 5

Sodium 6β-(p-bromobenzenesulphonyloxy)penicillanate

To a solution of benzyl 6β-hydroxypenicillanate (1.2 g) in pyridine (20 ml) was added p-bromobenzenesulphonyl chloride (2 g). The solution was allowed to stand at 4°C for 24 hr. then poured on to iced water and extracted with ethyl acetate (2 x 20 ml). The ethyl acetate layer was then washed with dilute sulphuric acid (2 x 100 ml) and dried over anhydrous sodium sulphate — sodium carbonate. Removal of the solvent in vacuo yielded benzyl 6β-(p-bromobenzenesulphonyloxy)-penicillanate as an orange brown solid which was purified by reprecipitation from ethyl acetate at −70°C by the addition of light petrol, to give an off white solid (1.6 g).

Benzyl 6β-(p-bromobenzenesulphonyloxy)penicillanate (0.6 g) was dissolved in tetrahydrofuran (15 ml) containing water (3 drops) and 10% palladium-carbon catalyst (1.2 g). The mixture was hydrogenolysed at atmospheric pressure for ½ hr then filtered (celite) and the solvent removed. The resulting oil was partitioned between ethyl acetate:water and neutralised with sodium hydroxide (0.5 M). The aqueous layer was separated and the water removed under reduced pressure to yield a pale brown solid (0.36 g), $\nu_{max}$ (KBr), 1775, 1610, 1090 and 890 $cm^{-1}$; δ ($D_2O$) 7.80 (4H, m, Ar*H*) 5.90 (1H, d, *J* = 4Hz, *H*—$C_6$), 5.40 (1H, d, *J* = 4Hz, *H*—$C_5$), 4.19 (1H, s, *H*—$C_3$), 1.55 (3H, s, gem $CH_3$), 1.40 (3H, s, gem $CH_3$).

## Example 6

Sodium 6β-(p-toluenesulphonyloxy)penicillanate 1,1-dioxide

To a solution of benzyl 6β-hydroxypenicillanate 1,1-dioxide (0.5 g) in pyridine (15 ml) at 0°C was added p-toluenesulphonyl chloride (0.6 g). The solution was allowed to stand at 4°C for 24 hr then poured on to iced water and extracted with ethyl acetate (2 x 20 ml). The ethyl acetate layer was washed with dilute sulphuric acid (2 x 30 ml) and finally dried over sodium sulphate — sodium carbonate. Removal of the solvent in vacuo yielded benzyl 6β-(p-toluenesulphonyloxy)penicillanate 1,1-dioxide as a pale yellow oil (0.58 g), $\nu_{max}$ ($CHCl_3$), 1820, 1750, 1600 $cm^{-1}$; δ ($CDCl_3$) 7.88 (2H, m, Ar*H*), 7.35 (7H, m, Ar*H*), 5.80 (1H, d, *J* = 4Hz, *H*—$C_6$), 5.20 (2H, q, $CH_2$—Ph), 4.75 (1H, d, J = 4Hz, *H*—$C_5$), 4.50 (1H, s, *H*—$C_3$), 2.44 (3H, s, Ar$CH_3$), 1.23 (3H, s, gem $CH_3$).

Benzyl 6β-(p-toluenesulphonyloxy)penicillanate 1,1-dioxide (0.2 g) was dissolved in tetrahydrofuran (10 ml) containing water (3 drops) and 10% palladium-carbon catalyst (0.4 g). The mixture was hydrogenolysed at atmospheric pressure for ½ hr then filtered (celite). Removal of the solvent in vacuo from the filtrate yielded a colourless glass which was dissolved in ethyl acetate (1 ml) to which solution 2M sodium 2-ethyl hexanoate in methyl isobutyl ketone (0.13 ml) was added. Sodium 6β-(p-toluenesulphonyloxy)penicillanate 1,1-dioxide was precipitated from the solution by addition of diethyl ether (20 ml) as a white solid (0.14 g), $\nu_{max}$ (KBr), 1800, 1620, 1460 and 1180

$cm^{-1}$; δ ($D_2O$) 7.60 (4H, m, Ar*H*), 6.03 (1H, d, *J* = 4Hz, *H*—$C_6$), 5.10 (1H, d, *J* = 4Hz, *H*—$C_5$), 4.32 (1H, s, *H*—$C_3$), 2.39 (3H, s, Ar$CH_3$), 1.50 (3H, s, gem $CH_3$), 1.35 (3H, s, gem $CH_3$).

Example 7

Lithium 6β-(p-toluenesulphonyloxy)penicillanate

Benzyl 6β-hydroxypenicillanate (0.202 g) was dissolved in chloroform (4 ml) containing dimethylformamide (0.15 ml). To this stirred solution at 0°C was added p-toluenesulphonyl chloride (0.087 g) and triethylamine (0.092 ml). The reaction mixture was allowed to warm to room temperature. After 48 hours, the reaction mixture was diluted with chloroform (20 ml), washed with dilute sodium bicarbonate (20 ml), washed with water (20 ml), dried ($MgSO_4$), and evaporated in vacuo to afford an oil. This oil was subjected to column chromatography on silica gel (20 g) using ethyl acetate:cyclohexane (1:3). The appropriate fractions were combined and evaporated in vacuo to afford the title compound as a gum, which was crystallised from ethyl acetate-cyclohexane to yield benzyl 6β-(p-toluenesulphonyloxy)penicillanate as white crystals (0.09 g) m.p. 97°C; i.r. (Nujol) 1790, 1757 $cm^{-1}$; n.m.r. ($CDCl_3$) 1.37 (3H, s), 1.57 (3H, s), 2.45 (3H, s), 4.46 (1H, s), 5.16 (2H, s), 5.45 (1H, d, *J* = 4Hz), 5.62 (1H, d *J* = 4Hz), 7.35 (5H, s), 7.60 (4H, ABq *J* = 9Hz).

Benzyl 6β-(p-toluenesulphonyloxy)penicillanate (0.078 g) in tetrahydrofuran (15 ml) was hydrogenated over 10% palladium-carbon catalyst (0.12 g) for 4 hr. The mixture was then filtered (celite), evaporated to 1 ml and water (5 ml) added. The resulting solution was titrated to pH 7.0 with LiOH and the solvent removed to yield, after washing with ether, lithium 6β-(p-toluenesulphonyloxy)penicillanate as a pinkish white solid (0.055 g), $\nu_{max}$ (KBr), 1780, 1610, 1195, 1180 $cm^{-1}$; ($D_2O$) 7.75 (2H, d, *J* = 8Hz, Ar*H*), 7.34 (2H, d, *J* = 8Hz, Ar*H*), 5.84 (1H, d, *J* = 3Hz, *H*—$C_6$), 5.37 (1H, d, J = 3Hz, *H*—$C_5$), 4.19 (1H, s, *H*—$C_3$), 2.32 (3H, s, Ar*H*), 1.49 (3H, s, gem $CH_3$), 1.39 (3H, s, gem $CH_3$).

Example 8

Sodium 6β-(p-nitrobenzenesulphonyloxy)penicillanate

To a solution of sodium 6β-hydroxypenicillanate (0.78 g) in dimethylformamide was added methoxymethyl chloride (0.65 ml). The solution was stirred at 0°C for 15 min then poured on to iced water and extracted with ethyl acetate (3 x 30 ml). The ethyl acetate layer was separated, washed with water, dried over anhydrous magnesium sulphate, and the solvent removed in vacuo. The resulting oil was chromatographed on silica gel (ethylacetate:petrol) to yield methoxymethyl 6β-hydroxypenicillanate (0.51 g), $\nu_{max}$ ($CHCl_3$) 1790, 1760, 1400, 1160 $cm^{-1}$, δ ($CDCl_3$) 5.58 (1H, d, *J* = 4Hz, *H*—$C_6$), 5.13 (1H, d, *J* = 4Hz, *H*—$C_5$), 5.30 (2H, s, $CH_2O$), 3.49 (3H, s, $CH_3O$), 1.67 (3H, s, gem $CH_3$), 1.55 (3H, s, gem $CH_3$).

Methoxymethyl 6β-hydroxypenicillanate (0.46 g) was dissolved in pyridine (2 ml) and p-nitrobenzenesulphonylchloride (0.4 g) added. The solution was allowed to stand at 4°C for 24 hr., then poured onto iced water and extracted with ethyl acetate (2 x 20 ml). The ethyl acetate layer was separated, washed with dilute sulphuric acid (2 x 40 ml) and dried over anhydrous sodium sulphate — sodium carbonate. Removal of the solvent in vacuo yielded methoxymethyl 6β-(p-nitrobenzenesulphonyloxy)penicillanate (0.7 g) as a pale brown oil, $\nu_{max}$ ($CHCl_3$) 1800, 1750, 1540, 1350, 1190 $cm^{-1}$; δ ($CDCl_3$) 8.43 (2H, d, *J* = 9 Hz, Ar*H*), 8.13 (2H, d, *J* = 9. Hz, Ar*H*), 5.78 (1H, d, *J* = 4 Hz, *H*—$C_6$), 5.53 (1H, d, *J* = 4 Hz, *H*—$C_5$), 5.28 (2H, s, $CH_2O$), 4.45 (1H, s, *H*—$C_3$), 3.45 (3H, s, O$CH_3$), 1.60 (3H, s, gem $CH_3$), 1.50 (3H, s, gem $CH_3$).

To a solution of methoxymethyl 6β-(p-nitrobenzenesulphonyloxy)penicillanate (0.18 g) in dimethylformamide was added lithium bromide (0.3 g). The solution was stirred overnight, then poured onto iced water and extracted with ethyl acetate (3 x 30 ml). The ethyl acetate layer was separated, washed with water and dried over anhydrous magnesium sulphate. The solution was then reduced to a small volume (5 ml), water (5 ml) was added and the pH was adjusted to 7.5 with sodium hydroxide. The aqueous layer was separated and the water removed in vacuo to yield a pale yellow glass (0.13 g) which on silica gel chromatography using butanol:ethanol:water (4:1:1) as eluant yielded sodium 6β-(p-nitrobenzenesulphonyloxy)penicillanate (0.07 g) as a pale yellow glass, $\nu_{max}$ (KBr) 1780, 1605, 1535, 1350, 1190 $cm^{-1}$; δ ($D_2O$) 8.34 (2H, d, *J* = 9 Hz, Ar*H*), 8.05 (2H, d, *J* = 9 Hz, Ar*H*), 5.88 (1H, d, *J* = 4 Hz, *H*—$C_6$), 5.43 (1H, d, *J* = 4 Hz, *H*—$C_5$), 4.16 (1H, s, *H*—$C_3$), 1.43 (3H, s, gem $CH_3$), 1.35 (3H, s, gem $CH_3$).

Example 9

Composition

a) A solution for injection may be prepared by dissolving 100 mg of sterile sodium 6β-methanesulphonyloxypenicillanate and 250 mg of sterile sodium amoxycillin in 1 ml of sterile water.

b) A solution for injection may be prepared by dissolving 125 mg of sterile sodium 6β-methanesulphonyloxypenicillanate and 125 mg of sterile cephaloridine in 1.5 ml of sterile water.

Demonstration 1

Sodium 6β-methanesulphonyloxypenicillanate

Benzyl 6β-hydroxypenicillanate (0.202 g) was dissolved in chloroform (4 ml) containing dimethylformamide (0.15 ml). To this stirred solution at 0°C, was added methanesulphonyl chloride (0.052 ml) and triethylamine (0.092 ml). The reaction mixture was allowed to warm to room temperature. After 20 hours, the reaction mixture was diluted with chloroform (20 ml), washed with dilute sodium bicarbonate (20 ml), washed with water (20 ml), dried ($MgSO_4$), and evaporated in vacuo to afford an oil. This oil was subjected to column chromatography on silica gel (25 g) using ethyl acetate:cyclohexane (1:3). The appropriate fractions were combined and evaporated in vacuo to afford benzyl 6β-methanesulphonyloxypenicillanate as an oil (0.11 g); i.r. (liq film) 1795, 1740 $cm^{-1}$; n.m.r. ($CDCl_3$) 1.41 (3H, s), 1.64 (3H, s), 3.18 (3H, s), 4.51 (1H, s), 5.19 (2H, s), 5.57 (1H, d $J$=4Hz), 5.76 (1H, d $J$=4 Hz), 7.36 (5H, s).

Benzyl 6β-methanesulphonyloxypenicillanate (0.10 g) was dissolved in a solvent mixture of tetrahydrofuran (3 ml), methanol (3 ml), and water (3 ml), and was hydrogenated over 5% Palladium on calcium carbonate (0.25 g) for 3 hours. The catalyst was filtered off and washed liberally with water and methanol. The combined filtrates were concentrated in vacuo to approximately 20 ml, and passed through an Amberlite IR—120 ($Na^{\oplus}$) ion-exchange column. The eluent was co-evaporated with n-propanol to afford the title compound as a white solid (0.065 g), i.r. (KBr) 3400 (broad), 1785, 1740 sh, 1650 sh, 1605 $cm^{-1}$; n.m.r. ($D_2O$) 1.48 (3H, s), 1.57 (3H, s), 3.30 (3H, s), 4.29 (1H, s), 5.52 (1H, d $J$=4Hz), 5.98 (1H, d $J$=4Hz).

Demonstration 2

Sodium 6β-methanesulphonyloxypenicillanate

To a solution of benzyl 6β-hydroxypenicillanate (1.8 g) in dichloromethane (10 ml) containing triethylamine (1.5 ml) at −10° was added methanesulphonyl chloride (1.0 ml). The solution was stirred at −10°C for 15 min, then washed with iced water, dilute hydrochloric acid, sodium bicarbonate, brine and finally dried over anhydrous magnesium sulphate. Removal of the solvent in vacuo yielded benzyl 6β-methanesulphonyloxypenicillanate as a pale yellow oil (2.1 g).

Benzyl 6β-methanesulphonyloxypenicillanate (2.0 g) was dissolved in tetrahydrofuran (50 ml) containing water (3 drops) and 10% palladium-carbon catalyst (4 g). The mixture was hydrogenolysed at atmospheric pressure for $\frac{1}{2}$ hr then filtered (celite) and the solvent evaporated in vacuo. The resulting oil was partitioned between ethyl acetate:water and neutralised with sodium hydroxide (0.5 M). The aqueous layer was separated and freeze dried to afford sodium 6β-methanesulphonyloxypenicillanate as an off-white solid (1.2 g).

Demonstration 3

Sodium 6β-phenylmethanesulphonyloxypenicillanate

To a solution of benzyl 6β-hydroxypenicillanate (0.5 g) in dichloromethane (10 ml) containing triethylamine (0.4 ml) at −10°C was added phenylmethanesulphonyl chloride (0.3 g). The solution was stirred at −10°C for 15 min., then washed with iced water, dilute hydrochloric acid, sodium bicarbonate, brine and finally dried over anhydrous magnesium sulphate. Removal of the solvent in vacuo yielded benzyl 6β-phenylmethanesulphonyloxypenicillanate as a pale yellow oil (0.061 g), $\nu_{max}$ ($CHCl_3$), 1790, 1740, 1380, 1190, 890 and 840 $cm^{-1}$; δ ($CDCl_3$) 7.41 (10H, m, Ar$H$), 5.52 (1H, d, $J$ = 4Hz, $H$—$C_6$), 5.37 (1H, d, $J$ = 4Hz, $H$—$C_5$), 5.18 (2H, s, Ph—$CH_2$—S), 4.53 (3H, s, Ph—$CH$—O, and $H$—$C_3$), 1.60 (3H, s, gem $CH_3$), 1.43 (3H, s, gem $CH_3$).

Benzyl 6β-phenylmethanesulphonyloxypenicillanate (0.4 g) was dissolved in tetrahydrofuran (25 ml) containing 10% palladium-carbon catalyst (0.8 g) and water (3 drops). The mixture was hydrogenolysed at atmospheric pressure, for $\frac{1}{2}$ hr. then filtered (celite) and the solvent evaporated in vacuo. The resulting oil was partitioned between ethyl acetate:water and neutralised with sodium hydroxide (0.5 M). The aqueous layer was separated and the water removed in vacuo to yield sodium 6β-phenylmethanesulphonyloxypenicillanate as a white solid (0.3 g), $\nu_{max}$ (KBr) 1775, 1350, 1200, 1175, 890 and 840 $cm^{-1}$; δ ($D_2O$) 7.49 (5H, s, Ar$H$), 5.75 (1H, d, $J$ = 4Hz, H—$C_6$), 5.45 (1H, d, $J$ = 4Hz, H—$C_5$), 4.82 (2H, s, $CH_2$—Ph), 4.25 (1H, s, $H$—$C_3$), 1.55 (3H, s, gem $CH_3$), 1.45 (3H, s, gem $CH_3$), $[\alpha]_D^{20}$ = +167.8° (c 1.1 $H_2O$).

Demonstration 4

Sodium 6β-benzenesulphonyloxypenicillanate

To a solution of benzyl 6β-hydroxypenicillanate (1.0 g) in pyridine (20 ml) was added benzenesulphonyl chloride (0.5 ml). The solution was allowed to stand at 4°C for 24 hr then poured on to iced water and extracted with ethyl acetate (2 × 40 ml). The ethyl acetate layer was then washed with dilute sulphuric acid (2 × 40 ml) and dried over anhydrous sodium sulphate-sodium carbonate. Removal of the solvent in vacuo yielded benzyl 6β-benzenesulphonyloxypenicillanate as a pale yellow oil (1.04 g), $\nu_{max}$ ($CHCl_3$) 1790, 1740, 1190 $cm^{-1}$; δ ($CDCl_3$) 7.83 (5H, m, Ar$H$), 7.35 (5H, s, Ar$H$ ester), 5.65 (1H, d, J=3.5 Hz, $H$—$C_6$), 5.45 (1H, d, $J$=3.5 Hz, $H$—$C_5$), 5.18 (2H, s, $CH_2$—Ph), 4.47 (1H, s, $H$—$C_3$), 1.56 (3H, s, gem $CH_3$), 1.38 (3H, s, gem $CH_3$).

Benzyl 6β-benzenesulphonyloxypenicillanate (1.1 g) was dissolved in aqueous tetrahydrofuran (10%, 30 ml), containing 10% palladium-carbon catalyst (2.3 g). The mixture was hydrogenolysed for $\frac{1}{2}$ hr at atmospheric pressure then filtered (celite) and the solvent removed in vacuo to yield 6β-benzenesulphonyloxypenicillanic acid (0.8 g) as a pale yellow oil. The oil was dissolved in ethyl acetate (5 ml) and 2M sodium 2-ethylhexanoate in methyl isobutyl ketone (1 ml) was added. 6β-benzenesulphonyloxypenicillanic acid was precipitated as the sodium salt (on the addition of diethyl ether (50 ml)) in the form of a red brown solid which crystallised on addition of water as colourless needles, $\nu_{max}$ (KBr), 1790, 1600, 1190 and 855 $cm^{-1}$; δ ($d_6$—DMSO) 7.85 (5H, m, Ar*H*), 5.97 (1H, d, $J$ = 3.5 Hz, *H*—$C_6$), 5.33 (1H, d, $J$ = 3.5 Hz, *H*—$C_5$), 4.18 (1H, s, *H*—$C_3$), 1.45 (3H, s, gem C$H_3$), 1.40 (3H, s, gem C$H_3$), $[\alpha]_D^{20}$ = +157.3° (c. 0.67 $H_2O$).

## Demonstration 5

Sodium 6β-(p-aminobenzenesulphonyloxy)penicillanate

To a solution of benzyl 6β-hydroxypenicillanate (0.5 g) in pyridine (10 ml) was aded p-nitrobenzenesulphonyl chloride (0.6 g). The solution was allowed to stand for 24 hr. at 4°C, then poured on to iced water and extracted with ethyl acetate (2 × 20 ml). The ethyl acetate layer was washed with dilute sulphuric acid (2 × 40 ml) and dried over anhydrous sodium sulphate-sodium carbonate. Removal of the solvent yielded benzyl 6β-(p-nitrobenzenesulphonyloxy)penicillanate as a pale yellow solid (0.5 g), $\nu_{max}$ ($CHCl_3$) 1800, 1740, 1600, 1530, 1190 cm−1; δ ($CDCl_3$) 8.40 (2H, d, $J$ = 9 Hz, Ar*H*), 8.13 (2H, d, $J$ = 9 Hz, Ar*H*), 7.35 (5H, s, $CH_2$—Ph), 5.75 (1H, d, $J$ = 4 Hz, *H*—$C_6$), 5.52 (1H, d, $J$ = 4 Hz, *H*—$C_5$), 5.16 (2H, s, C$H_2$—Ph), 4.47 (1H, s, *H*—$C_3$), 1.53 (3H, s, gem C$H_3$), 1.36 (3H, s, gem C$H_3$).

Benzyl 6β-(p-nitrobenzenesulphonyloxy)penicillanate (0.37) was dissolved in aqueous tetrahydrofuran (20 ml) containing 10% palladium-carbon catalyst (0.7 g). The mixture was hydrogenolysed at atmospheric pressure, until hydrogen uptake ceased, then filtered (celite) and removed in vacuo. The resulting oil was partitioned between ethyl acetate:water and neutralised with sodium hydroxide (0.5 M). The aqueous layer was separated and the water removed in vacuo to yield a pale yellow-glass which after silica gel chromatography (butanol:ethanol:water) (4:1:1) yielded sodium 6β-(p-aminobenzenesulphonyloxy)penicillanate as an off-white (0.1 g), $\nu_{max}$ (KBr) 1775, 1660, 1595, 1500, 1200, 1170 $cm^{-1}$; δ ($D_2O$) 7.63 (2H, d, $J$ = 9 Hz, Ar*H*), 6.76 (2H, d, $J$ = 9 Hz, Ar*H*), 5.74 (1H, d, $J$ = 4 Hz, *H*—$C_6$), 5.32 (1H, d, $J$ = 4 Hz, *H*—$C_5$), 4.15 (1H, s, *H*—$C_3$), 1.47 (3H, s, gem C$H_3$), 1.35 (3H, s, gem C$H_3$).

## Demonstration of effectiveness

In standard MIC tests the activities of the compounds of Example 1 and Demonstration 1 alone, and in combination with ampicillin were determined against Staph Russell and Proteus C889.

| | | MIC Staph Russell | Proteus C889 |
|---|---|---|---|
| Ampicillin + Sodium 6β-methanesulphonyloxy-penicillanate | @ 20 μgml⁻¹ | — | 4 |
| " " " " | @ 5 μgml⁻¹ | inhib | 125 |
| " " " " | @ 1 μgml⁻¹ | 0.08 | — |
| Ampicillin alone | | 62.5 | 1000 |
| Sodium 6β-methanesulphonyloxy-penicillanate | alone | 4 | 62.5 |
| Ampicillin + Calcium 6β-p-bromobenzenesulphonyloxy-penicillanate | @ 20 μgml⁻¹ | inhib | 16* |
| " " " " | @ 5 μgml⁻¹ | 0.16 | — |
| " " " " | @ 1 μgml⁻¹ | 0.31 | — |
| Ampicillin alone | | 250 | >2000 |
| Calcium 6β-p-bromobenzene-sulphonyloxypenicillanate | alone | 16 | >500 |

A composition of sodium 6β-methanesulphonyloxypenicillanate and cephaloridine against 7 cephalosporinase — producing E. coli strains results in the following MIC's in a standard test.

| | Geometric Mean (MIC) | +/− 95% confidence limits |
|---|---|---|
| Cephaloridine alone | 152.38 | (294.62—78.81) |
| Cephaloridine + synergist (5 μg/ml) | 11.32 | (14.71—8.71) |
| Cephaloridine + synergist (20 μg/ml) | 4.36 | (9.12—2.08) |

## Claims

1. A pharmaceutical composition comprising:
(a) a pharmaceutically acceptable carrier;
(b) a compound of the formula (I):

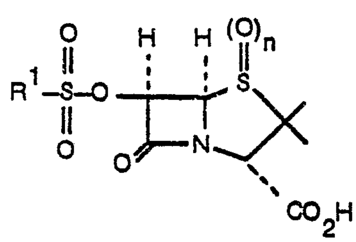

(I)

* trailing end-point

and pharmaceutically acceptably salts and in vivo hydrolysable esters thereof, wherein n is 0 or 2, $R^1$ is a $C_{1-6}$ alkyl group optionally substituted with one, two or three fluorine, chlorine or bromine atoms, or is a di-$C_{1-6}$ alkylamino group or is a group of the formula (i):

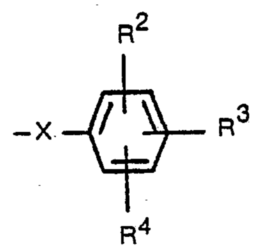

(i)

wherein

X is a bond or a —CH=CH-group, or a methylene or ethylene group; $R^2$ is a hydrogen, fluorine, bromine or chlorine atom, or is an amino, protected amino, hydroxy, protected hydroxy, $C_{1-6}$ alkyl, nitro, di-$C_{1-6}$ alkylamino, acetamido, $C_{1-6}$ alkoxy or trifluoromethyl group; $R^3$ is a hydrogen or chlorine atom or a $C_{1-6}$ alkyl or trifluoromethyl group; and $R^4$ is a hydrogen or chlorine atom, or a $C_{1-6}$ alkyl or $C_{1-6}$ alkoxy group; or $R^2$ and $R^3$ on any two adjacent carbon atoms may together represent a buta-1,3-dienylene moiety which is optionally substituted by a $C_{1-3}$ alkyl or $C_{1-3}$ alkoxy group; and

(c) a penicillin or cephalosporin or a pharmaceutically acceptable salt or in vivo hydrolysable ester thereof.

2. A composition as claimed in claim 1 wherein n is zero.

3. A composition as claimed in claim 2 wherein $R^1$ is a methyl or ethyl group.

4. A composition as claimed in claim 2 wherein $R^1$ is a group of the formula (iii):

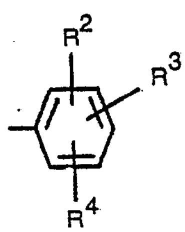

(iii)

wherein

$R^2$, $R^3$ and $R^4$ are as defined in relation to formula (I).

5. A composition as claimed in claim 4 wherein $R^3$ and $R^4$ are both hydrogen atoms and $R^2$ is selected from hydrogen, bromine, fluorine, chlorine, $C_{1-6}$ alkyl, nitro and amino.

6. A composition as claimed in any of claims 1—5 wherein the compound of the formula (I) is in the form of a sodium, potassium or calcium salt.

7. A composition as claimed in any of claims 1—6 wherein the penicillin is amoxycillin.

8. A composition as claimed in any of claims 1—6 wherein the cephalosporin is cephaloridine.

9. A compound of the formula (I) as depicted in claim 1, or pharmaceutically acceptable salt or in vivo hydrolysable ester thereof with the proviso that when n is zero, $R^1$ is not methyl, benzyl, p-chlorobenzyl, phenyl or p-aminophenyl when the compounds depicted in claim 1 are in the form of a free acid or a pharmaceutically acceptable salt; with the additional proviso that when n is zero $R^1$ is not trifluoromethyl or trichloromethyl.

10. A process for the preparation of a compound as claimed in claim 9 or salt or ester thereof which process comprises the reaction of a compound of the formula (III), or a derivative thereof which allows sulphonation to take place:

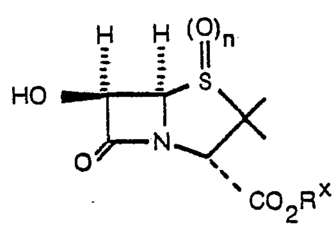

(III)

wherein

n is zero or 2 and $R^x$ is a hydrogen atom or a carboxy protecting group, with an O-sulphonating derivative of a compound of the formula (IV):

$$R^1SO_3H \quad (IV)$$

wherein

$R^1$ is as defined in claim 9; and thereafter if desired,

a) cleaving the carboxy protecting group to form the free acid or salt thereof.

b) converting the free acid or salt into a pharmaceutically acceptable salt or in vivo hydrolysable ester.

c) converting the compound wherein n is zero to the compound wherein n is 2.

**Patentansprüche**

1. Pharmazeutische Zusammensetzung, umfassend
(a) ein pharmakologisch verträgliches Trägermaterial,
(b) eine Verbindung der Formel (I)

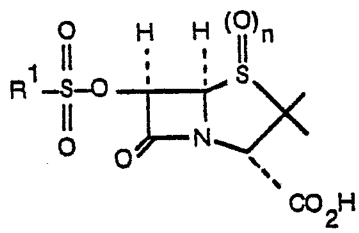

(I)

und deren pharmakologisch verträgliche Salz und in vivo hydrolysierbare Ester, in der n Null oder 2 ist,

$R^1$ ein $C_{1-6}$-Alkylrest, der gegebenenfalls mit einem, zwei oder drei Fluor-, Chlor- oder Bromatomen substituiert ist, oder ein Di-$C_{1-6}$-Alkylaminorest oder ein Rest der Formel (i)

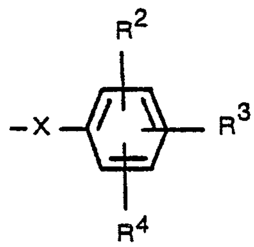

(i)

ist, wobei

X eine Bindung oder eine —CH=CH-Gruppe oder eine Methylen-oder Äthylengruppe ist,

$R^2$ ein Wasserstoff-, Fluor-, Brom- oder Chloratom oder eine Amino-, geschützte Amino-, Hydroxy-, geschützte Hydroxy-, $C_{1-6}$-Alkyl-, Nitro-, Di-$C_{1-6}$-alkylamino-, Acetamido-, $C_{1-6}$-Alkoxy- oder Trifluormethylgruppe ist,

$R^3$ ein Wasserstoff- oder Chloratom oder eine $C_{1-6}$-Alkyl- oder Trifluormethylgruppe ist und

$R^4$ ein Wasserstoff- oder Chloratom oder eine $C_{1-6}$-Alkyl- oder $C_{1-6}$-Alkoxygruppe ist oder

$R^2$ und $R^3$ an zwei beliebigen benachbarten Kohlenstoffatomen zusammen einen Buta-1,3-dienylen-Rest darstellen können, der gegebenenfalls durch einen $C_{1-3}$-Alkyl- oder $C_{1-3}$-Alkoxyrest substituiert ist, und

(c) ein Penicillin oder Cephalosporin oder dessen pharmakologisch verträgliches Salz oder in vivo hydrolysierbarer Ester.

2. Zusammensetzung nach Anspruch 1, wobei n Null ist.

3. Zusammensetzung nach Anspruch 2, wobei $R^1$ eine Methyl- oder Äthylgruppe ist.

4. Zusammensetzung nach Anspruch 2, wobei $R^1$ ein Rest der Formel (iii)

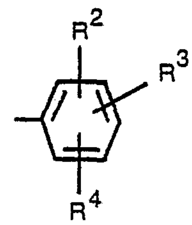

(iii)

ist, in der $R^2$, $R^3$ und $R^4$ in bezug auf die Formel (I) definiert sind.

5. Zusammensetzung nach Anspruch 4, wobei $R^3$ und $R^4$ Wasserstoffatome sind und $R^2$ aus Wasserstoff, Brom, Fluor, Chlor, $C_{1-6}$-Alkyl, Nitro und Amino ausgewählt ist.

6. Zusammensetzung nach einem der Ansprüche 1 bis 5, wobei die Verbindung der Formel (I) in der Form eines Natrium-, Kalium- oder Calciumsalzes vorliegt.

7. Zusammensetzung nach einem der Ansprüche 1 bis 6, wobei das Penicillin Amoxycillin ist.

8. Zusammensetzung nach einem der Ansprüche 1 bis 6, wobei das Cephalosporin Cephaloridin ist.

9. Verbindung der Formel (I) wie in Anspruch 1 dargestellt oder deren pharmakologisch verträgliches Salz oder in vivo hydrolysierbarer Ester mit der Maßgabe, daß, wenn n Null ist, $R^1$ kein Methyl, Benzyl, p-Chlorbenzyl, Phenyl oder p-Aminophenyl ist, wenn die in Anspruch 1 dargestellten Verbindungen in Form einer freien Säure oder eines pharmakologisch verträglichen Salzes vorliegen, und mit der weiteren Maßgabe, daß, wenn n Null ist, $R^1$ kein Trifluormethyl oder Trichlormethyl ist.

10. Verfahren zur Herstellung einer Verbindung nach Anspruch 9 oder deren Salz oder Ester, umfassend die Umsetzung einer Verbindung der Formel (III)

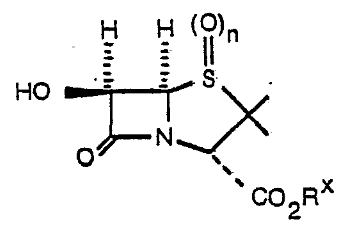

(III)

oder deren Derivat, das das Stattfinden einer Sulfonierung erlaubt, in der

n Null oder 2 ist und

$R^x$ ein Wasserstoffatom oder eine Carboxyschutzgruppe ist, mit einem O-Sulfonierungsderivat einer Verbindung der Formel (IV)

$$R^1SO_3H \qquad (IV)$$

in der $R^1$ wie in Anspruch 9 definiert ist, und danach gegebenenfalls

(a) Abspalten der Carboxyschutzgruppe zur Bildung der freien Säure oder deren Salz;

(b) Überführen der freien Säure oder des Salzes in ein pharmakologisch verträgliches Salz oder in einen in vivo hydrolysierbaren Ester;

(c) Überführen der Verbindung, bei der n Null ist, in eine Verbindung, in der n 2 ist.

## Revendications

1. Composition pharmaceutique renfermant:

(a) un véhicule ou excipient pharmaceutiquement acceptable;

(b) un composé de formule (I):

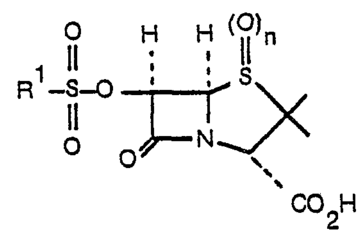

(I)

et des sels pharmaceutiquement acceptables et des esters hydrolysables in-vivo de celui-ci, formule dans laquelle n est 0 ou 2, $R^1$ est un groupe alcoyle en $C_{1-6}$ facultativement substitué par 1, 2 ou 3 atomes de fluor, de chlore ou de brome, ou est un groupe di-alcoyl($C_{1-6}$)amino, ou bien est un groupe de formule (i):

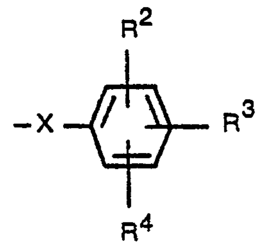

(i)

dans laquelle X est une liaison ou un groupe —CH=CH—, ou bien un groupe méthylène ou éthylène; $R^2$ est un atome d'hydrogène, de fluor, de brome ou de chlore ou est un groupe amino, amino protégé, hydroxy, hydroxy protégé, alcoyle en $C_{1-6}$, nitro, di-alcoyl($C_{1-6}$)amino, acétamido, alcoxy en $C_{1-6}$ ou trifluorométhyle; $R^3$ est un atome d'hydrogène ou de chlore ou un groupe alcoyle en $C_{1-6}$ ou trifluorométhyle; et $R^4$ est un atome d'hydrogène ou de chlore, ou un groupe alcoyle en $C_{1-6}$ ou alcoxy en $C_{1-6}$; ou bien $R^2$ et $R^3$ fixés sur deux atomes de carbone adjacents quelconques peuvent représenter ensemble une fraction molaire buta-1,3-diénylène qui est facultativement substituée par un groupe alcoyle en $C_{1-3}$ ou alcoxy en $C_{1-3}$; et

(c) une pénicilline ou une céphalosporine ou un sel pharmaceutiquement acceptable ou un ester hydrolysable in-vivo de celle-ci.

2. Composition suivant la revendication 1, dans laquelle n est égal à 0.

3. Composition suivant la revendication 2, dans laquelle $R^1$ est un groupe méthyle ou éthyle.

4. Composition suivant la revendication 2, dans laquelle $R^1$ est un groupe de formule (iii):

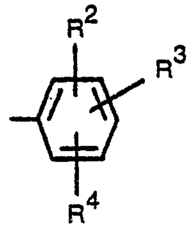

(iii)

où $R^2$, $R^3$ et $R^4$ sont tels que définis dans le cas de la formule (I).

5. Composition suivant la revendication 4, dans laquelle $R^3$ et $R^4$ sont tous deux des atomes d'hydrogène et $R^2$ est choisi parmi l'hydrogène, le brome, le fluor, le chlore ou un groupe alcoyle en $C_{1-6}$, nitro ou amino.

6. Composition suivant l'une quelconque des revendications 1 à 5, dans laquelle le composé de formule (I) est sous la forme d'un sel de sodium, de potassium ou de calcium.

7. Composition suivant l'une quelconque des revendications 1 à 6, dans laquelle la pénicilline est de l'amoxicilline.

8. Composition suivant l'une quelconque des revendications 1 à 6, dans laquelle la céphalosporine est de la céphaloridine.

9. Composé de formule (I) suivant la revendication 1 ou sel pharmaceutiquement acceptable ou ester hydrolysable in-vivo de ce composé, à condition que lorsque n est égal à 0, $R^1$ ne soit pas un groupe méthyle, benzyle, p-chlorobenzyle, phényle ou p-aminophényle quand les composés visés par la revendication 1 sont sous la forme d'un acide libre ou d'un sel pharmaceutiquement acceptable, et à condition en outre que, lorsque n est égal à 0, $R^1$ ne soit pas un groupe trifluorométhyle ou trichlorométhyle.

10. Procédé pour la préparation d'un composé suivant la revendication 9 ou d'un sel ou ester de celui-ci, ce procédé consistant à effectuer la réaction d'un composé de formule (III) ou d'un dérivé ce celui-ci permettant à la sulfonation de s'effectuer:

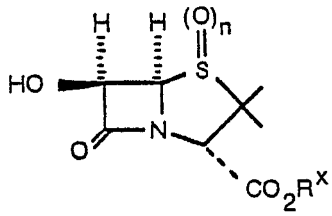

(III)

dans laquelle n est égal à 0 ou 2 et $R^x$ est un atome d'hydrogène ou un groupe de protection du carboxyle, avec un dérivé de sulfonation sur 0 d'un composé de formule (IV):

$$R^1SO_3H \qquad (IV)$$

dans laquelle $R^1$ a la même signification que dans le cas de la revendication 9, puis si désiré:

(a) à séparer le groupe de protection du carboxyle pour former l'acide libre ou un sel de celui-ci;

(b) à convertir l'acide libre ou son sel en un sel pharmaceutiquement acceptable ou ester hydrolysable in-vivo;

(c) à convertir le composé dans lequel n est égal à 0 en composé dans lequel n est égal à 2.